# EUROPÄISCHE PATENTANMELDUNG

(11) **EP 4 249 600 A1**
(43) Veröffentlichungstag der Anmeldung: **27.09.2023**
(21) Anmeldenummer: 22164131.9
(22) Anmeldetag: 24.03.2022
(51) Int. Cl.: C12P 19/00, C12P 19/04, C12P 19/14, A23C 9/12, A23L 33/21

(54) **GALACTOOLIGOSACCHARID-ZUBEREITUNGEN MIT GELFÖRMIGER KONSISTENZ**

(71) Anmelder: DMK Deutsches Milchkontor GmbH, 27404 Zeven (DE)
(72) Erfinder: DÖRING, Sven-Rainer, 27404 Zeven (DE); Steffens, Marco, 27404 Elsdorf (DE)
(74) Vertreter: Fabry, Bernd

(57) **Zusammenfassung**

Vorgeschlagen werden Galactooligosaccharidzubereitungen mit gelförmiger Konsistenz, erhalten oder erhältlich durch die folgenden Schritte:
(a) Bereitstellen einer wässrigen Milchzuckerlösung basierend auf Magermilch;
(b) Konzentrierung der Milchzuckerlösung aus Schritt (i);
(c) Entkeimung der Milchzuckerlösung aus Schritt (ii) durch Pasteurisierung;
(d) Transgalactosilierung der in der entkeimten Milchzuckerlösung aus Schritt (iii) vorhandenen Milchzucker unter Zugabe mindestens einer beta-Galactosidase innerhalb deren optimalen Temperatur- und pH-Wert-Intervalls über einen Zeitraum von mindestens 30 Minuten unter Erhalt einer Reaktionsmischung,
(e) Desaktivierung der Enzymmasse in der Reaktionsmischung aus Schritt (d) durch thermische Behandlung und
(f) Konfektionierung des so erhaltenen Produktes.

## Beschreibung

### GEBIET DER ERFINDUNG

Die vorliegende Patentanmeldung betrifft ein Produkt und ein Verfahren zur Herstellung von speziellen Nahrungsergänzungsmitteln bestehend aus oligomeren Kohlenhydraten und Proteinen basierend auf Magermilch.

### TECHNOLOGISCHER HINTERGRUND

Galactooligosaccharide (GOS) 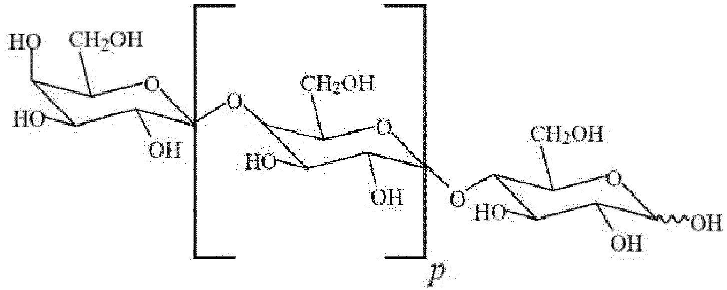 auch bekannt als Oligogalaktosyllactose, Oligogalaktose, Oligolaktose oder Transgalaktooligosaccharide (TOS), gehören zur Gruppe der Präbiotika. GOS kommt in handelsüblichen Produkten wie Nahrung für Säuglinge und Erwachsene vor.

Aufgrund der Konfiguration ihrer glykosidischen Bindungen widerstehen Galactooligosaccharide (GOS) weitgehend der Hydrolyse durch Speichel- und Darmverdauungsenzyme. Galactooligosaccharide werden daher als Präbiotika eingestuft, definiert als unverdauliche Nahrungsbestandteile, die sich vorteilhaft auf den Wirt auswirken, indem sie das Wachstum und/oder die Aktivität nützlicher Bakterien im Dickdarm stimulieren. Die erhöhte Aktivität dieser gesundheitsfördernden Bakterien führt zu einer Reihe von Effekten, sowohl direkt durch die Bakterien selbst als auch indirekt durch die organischen Säuren, die sie durch Fermentation produzieren. Beispiele für Wirkungen sind die Stimulierung der Immunfunktionen, die Aufnahme essentieller Nährstoffe, und die Synthese bestimmter Vitamine.

Galactooligosaccharide sind ein Substrat für Bakterien, wie Bifidobakterien und Laktobazillen. Studien mit Säuglingen und Erwachsenen haben gezeigt, dass mit Galakto-Oligosacchariden angereicherte Lebensmittel oder Getränke zu einer signifikanten Zunahme von Bifidobakterien führen. Diese Zucker kommen natürlich in der menschlichen Milch vor und sind als Oligosaccharide der menschlichen Milch bekannt. Beispiele sind Lakto-N-Tetraose, Lakto-N-Notetraose und Lakto-N-Fucopentaose.

Die menschliche Darmmikrobiota spielt eine Schlüsselrolle im intestinalen Immunsystem. Galacto-Oligosaccharide unterstützen die natürlichen Abwehrkräfte des menschlichen Körpers über die Darmmikroflora, indirekt, indem sie die Anzahl der Bakterien im Darm erhöhen und die Bindung oder das Überleben von *Escherichia coli, Salmonella Typhimurium* und Clostridien hemmen. GOS können das Immunsystem indirekt durch die Produktion von antimikrobiellen Substanzen positiv beeinflussen, indem sie die Vermehrung von pathogenen Bakterien reduzieren. Verstopfung ist ein potenzielles Problem, insbesondere bei Säuglingen, älteren Menschen und schwangeren Frauen. Bei Säuglingen kann die Fütterung mit Säuglingsnahrung mit Verstopfung und hartem Stuhl assoziiert sein. GOS können die Stuhlfrequenz verbessern und die mit der Verstopfung verbundenen Symptome lindern.

Die typische Gewinnung von GOS umfasst die folgenden Schritte:
1. Konzentrierung einer Milchzuckerlösung (Lactose, Sauermolke, Milchpermeat mit dem Ziel eine Trockenmasse von mindestens 30 Gew.-% zu erreichen;
2. Hocherhitzung/UHT-Behandlung zur Entkeimung bei 85-140 C über 90 bis 300 s;
3. Zugabe des Enzyms (z.B.*Aspergillus Oryzae)* und Einstellung der für das Enzym optimalen pH- und Temperaturbedingungen (z.B. pH =4,5, 55 C);
4. Verweilzeit in der Regel über 30 bis maximal 120 min (abhängig vom Enzym), da ansonsten eine Rückspaltung erfolgt;
5. Thermische Inaktivierung des Enzyms beispielsweise durch Hochtemperaturpasteurisierung (90 °C, 10 min)
6. Aufreinigung, gegebenenfalls Konzentrierung, Sprühtrocknung.
Eine Übersicht zur Herstellung von Galactooligosacchariden findet sich auch von K. Zerge in einer Online-Publikation der Universität Dresden: https://nbn-resolving.org/urn:nbn:de:bsz:14-qucosa-157121

### RELEVANTER STAND DER TECHNIK

EP 2620506 B1 (DUPONT) betrifft die Gewinnung von GOS ausgehend von Lactitol.

EP 3598901 B1 (HOCHSCHULE ANHALT) betrifft ein Verfahren zur Herstellung von GOS, bei dem man eine von L.bulgaricus (L.delbrueckii spp.bulgaricus) abgeleitete beta-Galactosidase bei einer Temperatur von 37 C oder weniger mit einer Lactose-haltigen Zusammensetzung wie Milch, Puffer oder Molke, z.B. Süßmolke, Sauermolke, Molkenkonzentrat oder Molkenpermeat, inkubiert.

EP 3041945 B1 (FRIESLAND) stellt ein Verfahren zur Herstellung von GOS aus Lactose bereit, das (i) das Inkontaktbringen einer Lactosebeschickung mit immobilisierter beta-Galactosidase (EC 3.2.1.23) und (ii) das Ermöglichen der GOS-Synthese umfasst, wobei die Lactosebeschickung eine wässrige Aufschlämmung von kristalliner Lactose ist.

WO 2008 037839 A1 (VALIO) bezieht sich auf ein Verfahren zur Herstellung von GOShaltigen Produkten auf Milchbasis durch Behandlung mit einer beta-Galactosidase.

WO 2018 048305 A1 (UNIV GRONINGEN) beschreibt die Verwendung einer GOS-Zusammensetzung, die verzweigte und lineare GOS-Spezies mit einem Polymerisationsgrad (DP) von 3 umfasst, wobei die verzweigten DP3-GOS-Spezies im Überschuss gegenüber den linearen DP3-GOS-Spezies vorhanden sind, zur Induktion von Mucin-Glykan-Verwertungswegen in nützlichen Darmbakterien in einem Tier.

WO 2018 210820 A1 (NOVOZYMES) beansprucht ein Verfahren, bei dem Milchsubstrat mit einem Laktosegehalt von mindestens 20 Gew.-% Laktose wird mit einem Enzym mit transgalaktosylierender Aktivität behandelt wird. Die transgalaktosylierende Aktivität des Enzyms kann durch Glykation von Lysin- und/oder Argininresten durch Inkubation des Enzyms mit hohen Glukosekonzentrationen bei erhöhten Temperaturen erhöht worden sein.

WO 2020 049016 A1 (FRIESLAND) bezieht sich auf das Gebiet der hypoallergenen Oligosaccharide zur Verwendung in Nahrungszusammensetzungen, insbesondere auf Oligosaccharide mit präbiotischen Eigenschaften. Es wird eine hypoallergene Oligosaccharid-Zusammensetzung bereitgestellt, die Galactooligosaccharide (GOS) umfasst, wobei (i) der Gehalt an Galactooligosacchariden (GOS) mindestens 40 Gew.-% der Gesamttrockensubstanz der Zusammensetzung beträgt; (ii) der Gehalt an Allolactose mindestens 10 Gew.-% der Gesamttrockensubstanz der Zusammensetzung beträgt; (iii) der Gehalt an 6'-GL mindestens 30 Gew.-% der Gesamt-GOS in der Zusammensetzung beträgt; und (iv) mindestens 0. 5 Gewichtsprozent des gesamten GOS einen Polymerisationsgrad (DP) von sechs oder mehr aufweisen.

WO 2020 117548 A1 (DUPONT) betrifft ein Verfahren zur Bereitstellung eines laktosearmen Produkts auf Milchbasis mit GOS-Fasern, bei dem ein Milchsubstrat mit Laktose mit einem transgalaktosylierenden Enzym behandelt wird, um GOS-Fasern und verbleibende Laktose bereitzustellen; Deaktivieren des transgalaktosylierenden Enzyms; Inkontaktbringen des Substrats auf Milchbasis mit GOS-Fasern mit einer Laktase, um die verbleibende Laktose abzubauen, um das laktosearme Produkt auf Milchbasis mit GOS-Fasern bereitzustellen, und Deaktivieren der Laktase.

WO 2020 141032 A1 (FRIESLAND) bezieht sich auf das Gebiet der Nahrungsmittelbestandteile, insbesondere auf wirtschaftlich attraktive Verfahren zur Herstellung von hypoallergenen Galactooligosacchariden (HA-GOS) und deren Verwendung in Nahrungs- und Futtermitteln. Es wird ein Verfahren zur Herstellung eines HA-GOS-Präparats bereitgestellt, das das Inkontaktbringen eines Lactose-Einsatzmaterials mit einer speziellen beta-Galactosidase (EC 3.2.1.23) umfasst, wobei das Lactose-Einsatzmaterial ein Käsemolkenpermeat (CWP) oder ein CWP ist, das mit Sialyllactose angereichert ist (SL-CWP).

### AUFGABE DER ERFINDUNG

Es besteht Bedarf an GOS-Zubereitungen als Nahrungsergänzungsmittel, welche zusätzlich zu einem hohen Kohlenhydratanteil auch einen hohen Proteinanteil aufweisen, jedoch kein Fett enthalten und daher kalorienarm sind. Außerdem besteht Bedarf an fett- und salzfreien GOS-Zubereitungen, die nicht oder nur minimal konfektioniert werden müssen, sondern direkt als fertiges Produkt mit hoher Haltbarkeit vertrieben werden können und somit besonders kostengünstig sind.

### BESCHREIBUNG DER ERFINDUNG

Ein erster Gegenstand der Erfindung betrifft daher Galactooligosaccharidzubereitungen mit gelförmiger Konsistenz, erhalten oder erhältlich durch die folgenden Schritte:
(a) Bereitstellen einer wässrigen Milchzuckerlösung basierend auf Magermilch;
(b) Konzentrierung der Milchzuckerlösung aus Schritt (a);
(c) Entkeimung der Milchzuckerlösung aus Schritt (b) durch Pasteurisierung;
(d) Transgalactosilierung der in der entkeimten Milchzuckerlösung aus Schritt (c) vorhandenen Milchzucker unter Zugabe mindestens einer beta-Galactosidase innerhalb deren optimalen Temperatur- und pH-Wert-Intervalls über einen Zeitraum von mindestens 30 Minuten unter Erhalt einer Reaktionsmischung,
(e) Desaktivierung der Enzymmasse in der Reaktionsmischung aus Schritt (d) durch thermische Behandlung und
(f) Konfektionierung des so erhaltenen Produktes.

Ein zweiter Gegenstand der Erfindung betrifft ein Verfahren zur Herstellung von Galactooligosaccharidzubereitungen mit gelförmiger Konsistenz, erhalten oder erhältlich durch die folgenden Schritte:
(a) Bereitstellen einer wässrigen Milchzuckerlösung basierend auf Magermilch;
(b) Konzentrierung der Milchzuckerlösung aus Schritt (a);
(c) Entkeimung der Milchzuckerlösung aus Schritt (b) durch Pasteurisierung;
(d) Transgalactosilierung der in der entkeimten Milchzuckerlösung aus Schritt (c) vorhandenen Milchzucker unter Zugabe mindestens einer beta-Galactosidase innerhalb deren optimalen Temperatur- und pH-Wert-Intervalls über einen Zeitraum von mindestens 30 Minuten unter Erhalt einer Reaktionsmischung,
(e) Desaktivierung der Enzymmasse in der Reaktionsmischung aus Schritt (d) durch thermische Behandlung und
(f) Konfektionierung des so erhaltenen Produktes.

Überraschenderweise wurde gefunden, dass bei der Desaktivierung des zugegebenen Enzyms während der Herstellung von GOS-Zubereitungen aus Magermilch oder Magermilchkonzentraten, ein starker Anstieg der Viskosität zu beobachten ist, wobei das Produkt die Textur eines gelierten Feststoffs annahm. Bei der Verwendung der bisherigen Ausgangsstoffe für GOS-Zubereitungen, welche keine Proteine enthalten, führt thermische Desaktivierung der beigefügten Enzyme nicht zu einer signifikanten Erhöhung der Viskosität. Auch bei Desaktivierung der Enzyme durch pH-Änderung wird der Effekt nicht beobachtet. Es handelt sich hier folglich um ein völlig neues Produkt, das kalorienarm, salz- und fettfrei ist, jedoch große Mengen and Proteinen sowie Galactooligosaccharide enthält. Außerdem erlaubt die thermische Desaktivierung sowie die daraus resultierende Veränderung der Viskosität, dass das entstehende Produkt ohne Weiterverarbeitung genutzt werden kann und eine hohe Haltbarkeit aufweist. Daher löst das Produkt sowie das Verfahren die eingangs geschilderte Aufgabe vollumfänglich. Insbesondere basiert das erfindungsgemäße Produkt auf einem wenig verarbeiteten natürlichen Produkt, nämlich Magermilch, und stellt ein für die menschliche und tierische Ernährung geeignete Zubereitung dar, welche effizient die nährreichen Bestandteile der Magermilch extrahiert und dabei kostengünstig in der Herstellung ist.

### Einsatzstoffe

Als Einsatzstoffe für die Herstellung von Galactooligosaccharidzubereitungen kommen ausschließlich proteinreiche Milchzuckerlösungen auf Basis von Magermilch oder Magermilchkonzentraten in Frage. Wesentlich und gemeinsam ist den geeigneten Einsatzstoffen, dass sie eine ausreichende Menge an Lactose, speziell an glykosidisch gebundener Galactose besitzen und zudem einen hohen Anteil an Proteinen aufweisen.

Lactose, Milchzucker oder Laktose ist ein in Milch enthaltener Zucker.

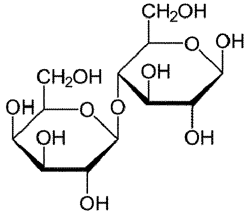

Das Disaccharid besteht aus den beiden Molekülen D-Galactose und D-Glucose, die über eine β-1,4-glycosidische Bindung miteinander verbunden sind. Nach IUPAC wird Lactose als 4-*O*-(β-D-Galactopyranosyl)-D-glucopyranose bezeichnet. Sie kommt als Hauptenergieträger in der Milch der Säugetiere vor. Lactose wird im Dünndarm vom Enzym Lactase verdaut, d. h. in Glucose und Galactose gespalten. In der Milch der Säugetiere sowie in Milcherzeugnissen macht Lactose fast den gesamten Anteil der Kohlenhydrate aus. Lactose, liefert Energie, unterstützt die Calcium-Resorption, hemmt Fäulnisbakterien im Darm des Menschen und begünstigt Bifidus-Bakterien (Bifidobacterium).

Molke ist die wässrige grünlich-gelbe Restflüssigkeit, die bei der Käseherstellung entsteht. Sie ist der flüssige Teil, der nach der Gerinnung der Milch zu Käse oder Quark abgesondert werden kann. Sauermolke entsteht, wenn Milch mit Milchsäurebakterien behandelt wird.

Es empfiehlt sich, Milchzuckerlösungen mit einer ausreichend hohen Feststoffmenge (synonym: Trockenmasse) einzusetzen, um das erfindungsgemäße Verfahren mit ökonomisch sinnvollen Umsätzen und Ausbeuten durchführen zu können. Hierzu eignen sich Lösungen, die eine Feststoffmenge von etwa 25 bis etwa 50 Gew.-% und vorzugsweise etwa 30 bis etwa 35 Gew.-% aufweisen. Gegebenenfalls können technische Milchzuckerlösungen beispielsweise durch Umkehrosmose ("reverse osmosis RO") entsprechend aufkonzentriert werden.

### Entkeimung

Im ersten Schritt des erfindungsgemäßen Verfahrens werden die wässrigen Milchzuckerlösungen entkeimt. Darunter ist jeder Prozess zu verstehen, mit dem sich die Keimlast des natürlichen Ausgangsproduktes auf einen Wert vermindern lässt, der unter dem liegt, den die jeweiligen nationalen Prüfstellen als Schwelle für die Zulassung als Lebensmittel festgelegt haben. In der Regel werden die Milchzuckerlösungen auf unter 1.000 Keime/mL entkeimt, vorzugsweise auf unter 500 Keime/mL und insbesondere etwa 10 bis etwa 50 Keime/mL. Die bevorzugte Entkeimungsmethode ist eine Hochtemperaturbehandlung, bei der die Lösungen einer Temperatur im Bereich von etwa 70 bis etwa 150 °C, vorzugsweise etwa 90 bis etwa 120 C über etwa 3 bis etwa 300 Sekunden, vorzugsweise etwa 50 bis etwa 200 Sekunden ausgesetzt werden. Eine Entkeimung kann entfallen, wenn bereits eine entkeimte Milchzuckerlösung eingesetzt wird.

### Thermische Desaktivierung von Enzymen

Die Transgalactosilierung wird vorzugsweise solange durchgeführt, bis die höchste GOS-Konzentration erreicht ist. Dieser durch Enzym und Reaktionsbedingungen bedingte Wert kann durch Probennahme verfolgt und damit vom Fachmann leicht ermittelt werden. Ist das Maximum der GOS-Bildung erreicht, muss die Aktivität der Enzyme sehr schnell gestoppt werden, um eine Rückspaltung zu vermeiden. Dies erfolgt durch schnelle Hocherhitzung, bei der das Enzymmaterial vollständig denaturiert wird.

Die Abtrennung der desaktivierten Enzyme ist optional und erfolgt vorzugsweise durch Filtration die vorzugsweise kontinuierlich durchgeführt wird.

### Konfektionierung

Das nach thermischer Desaktivierung und gegebenenfalls Abtrennung der desaktivieten Enzymmasse erhaltene Produkt weist eine gelartige Konsistenz auf und kann in dieser Form unmittelbar abgefüllt und in den Handel gebracht werden.,

Es ist aber grundsätzlich auch möglich, das Produkt zu trocknen und zuvor gegebenenfalls aufzureinigen und/oder zu konzentrieren. In einer besonderen Ausführungsform des erfindungsgemäßen Verfahrens kann das Produkt beispielsweise einer Nanofiltration unterworfen werden, bei der unerwünschte Monosaccharide in das Permeat gehen und die Menge an GOS im Retentat angereichert wird. Alternativ können die Monosaccharide auch mit Hefen versetzt werden, die Lactose zu Ethanol und Kohlendioxid verstoffwechseln. Die Hefen können anschließend durch beispielsweise in einem Separator oder Dekanter mit nachfolgender Filtration abgetrennt und in den Kreislauf zurückgeführt werden. Schließlich ist es ebenso möglich, den Restgehalt an Lactose durch Zugabe von Lactase zu vermindern.

Um die GOS-Konzentration zu erhöhen können die GOS-reichen Retentate noch aufgereinigt werden, beispielsweise durch Elektrodialyse oder Membranverfahren, wie z.B. eine Umkehrosmose. Falls erforderlich, kann die Trockenmasse durch Eindampfen erhöht werden.

Die Trocknung kann dann beispielsweise durch Lyophilisierung, vorzugsweise aber durch Sprühtrocknung erfolgen, wobei die Temperatur im Einlass typischerweise etwa 180 bis etwa 260 °C und am Ausgang etwa 80 bis etwa 105 °C beträgt. Der Restwassergehalt beträgt dabei maximal 5 Gew.-% und vorzugsweise etwa 1 bis etwa 2 Gew.-%.

### GEWERBLICHE ANWENDBARKEIT

Ein weiterer Gegenstand der vorliegenden Erfindung betrifft die Verwendung der Galactooligosaccharidzubereitungen als Nahrungsmittel, Nahrungsergänzungsmittel oder Futtermittel für die Tierernährung.

### BEISPIELE

### Beispiel 1

### Thermische Desaktivierung der Enzymmasse

kg einer Magermilch mit 30 Gew.-% Trockenmasse wurde in einem Röhrenwärmeaustauscher über 120 Sekunden auf 98 °C erhitzt und dabei entkeimt. Die entkeimte Lösung wurde auf 50 C abgekühlt, in einen Fermenter überführt, mit Hilfe von Milchsäure auf einen pH-Wert von 6,0 eingestellt, mit beta-Galactosidase *aus Bacillus circulans* im Gewichtsverhältnis Enzym:Substrat von 1:50 versetzt und gerührt. Der Fortschritt der Transgalactosilierung wurde durch Probennahme verfolgt. Nach etwa 15 Minuten wurde das Viskositätsmaximum erreicht. Die Reaktionsmischung wurde anschließend innerhalb von 30 Sekunden auf 98 C erhitzt und dort für weitere 60 Sekunden belassen. Schon während der Desaktivierung wurde ein fortschreitender anstieg der Viskosität festgestellt. Das resultierende Endprodukt zeigte eine gelförmige Konsistenz und ließ sich ohne Schwierigkeiten sowohl ausgießen als auch pumpen und konfektionieren.

### Vergleichsbeispiel V1

### Desaktivierung der Enzymmasse durch pH-Shift

kg einer Magermilch mit 30 Gew.-% Trockenmasse wurde in einem Röhrenwärmeaustauscher über 120 Sekunden auf 98 °C erhitzt und dabei entkeimt. Die entkeimte Lösung wurde auf 50 C abgekühlt, in einen Fermenter überführt, mit Hilfe von Milchsäure auf einen pH-Wert von 6,0 eingestellt, mit beta-Galactosidase *aus Bacillus circulans* im Gewichtsverhältnis Enzym:Substrat von 1:50 versetzt und gerührt. Der Fortschritt der Transgalactosilierung wurde durch Probennahme verfolgt. Nach etwa 15 Minuten wurde das Viskositätsmaximum erreicht. Der pH-Wert wurde durch Zugabe von 30 Gew.-%iger Natronlauge innerhalb von wenigen Minuten auf 10,0 eingestellt, wodurch die Aktivität des Enzyms schlagartig um 80 % verringert wurde. Es wurde kein Viskositätsanstieg beobachtet, das resultierende Produkt war flüssig und niedrigviskos.

### Vergleichsbeispiel V2

### Einsatz von Sauermolke

kg einer ca. 30 Gew.-%igen Sauermolke (pH = 5,1) wurde in einem Röhrenwärmeaustauscher über 120 Sekunden auf 98 °C erhitzt und dabei entkeimt. Die entkeimte Lösung wurde auf 50 C abgekühlt, in einen Fermenter überführt, mit beta-Galactosidase aus *Aspergillus oryzae* im Gewichtsverhältnis Enzym:Substrat von 1:50 versetzt und gerührt. Der Fortschritt der Transgalactosilierung wurde durch Probennahme verfolgt. Nach etwa 90 Minuten war die maximale GOS-Konzentration erreicht. Die Reaktionsmischung wurde anschließend innerhalb von 30 Sekunden auf 98 C erhitzt und dort für weitere 60 Sekunden belassen. Es wurde kein Viskositätsanstieg beobachtet, das resultierende Produkt war flüssig und niedrigviskos.

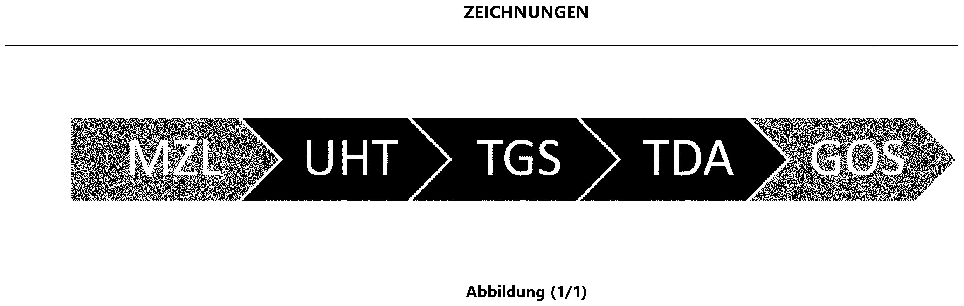

Der Prozess wird ferner an Hand eines Fließschemas gemäß **Abbildung 1** näher erläutert; dabei bedeuten die Abkürzungen:
- MZL: : Milchzuckerlösung
- UHT: : Ultrahocherhitzung
- TGS: : Transgalactosilierung
- TDA: : Thermische Desaktivierung
- GOS: : Galactooligosaccharide

## Patentansprüche

1. Galactooligosaccharidzubereitungen mit gelförmiger Konsistenz, erhalten oder erhältlich durch die folgenden Schritte:
(a) Bereitstellen einer wässrigen Milchzuckerlösung basierend auf Magermilch;
(b) Konzentrierung der Milchzuckerlösung aus Schritt (a);
(c) Entkeimung der Milchzuckerlösung aus Schritt (b) durch Pasteurisierung;
(d) Transgalactosilierung der in der entkeimten Milchzuckerlösung aus Schritt (c) vorhandenen Milchzucker unter Zugabe mindestens einer beta-Galactosidase innerhalb deren optimalen Temperatur- und pH-Wert-Intervalls über einen Zeitraum von mindestens 30 Minuten unter Erhalt einer Reaktionsmischung,
(e) Desaktivierung der Enzymmasse in der Reaktionsmischung aus Schritt (d) durch thermische Behandlung und
(f) Konfektionierung des so erhaltenen Produktes.

2. Verfahren zur Herstellung von Galactooligosaccharidzubereitungen mit gelförmiger Konsistenz, umfassend die folgenden Schritte:
(a) Bereitstellen einer wässrigen Milchzuckerlösung basierend auf Magermilch;
(b) Konzentrierung der Milchzuckerlösung aus Schritt (a);
(c) Entkeimung der Milchzuckerlösung aus Schritt (b) durch Pasteurisierung;
(d) Transgalactosilierung der in der entkeimten Milchzuckerlösung aus Schritt (c) vorhandenen Milchzucker unter Zugabe mindestens einer beta-Galactosidase innerhalb deren optimalen Temperatur- und pH-Wert-Intervalls über einen Zeitraum von mindestens 30 Minuten unter Erhalt einer Reaktionsmischung,
(e) Desaktivierung der Enzymmasse in der Reaktionsmischung aus Schritt (d) durch thermische Behandlung und
(f) Konfektionierung des so erhaltenen Produktes.

3. Verfahren nach mindestens einem der Ansprüche 2 bis 3, **dadurch gekennzeichnet, dass** man die Entkeimung durch eine Hochtemperaturbehandlung bewirkt.

4. Verfahren nach mindestens einem der Ansprüche 2 bis 4, **dadurch gekennzeichnet, dass** man die Pasteurisierung bei einer Temperatur im Bereich von etwa 70 bis etwa 150 °C und über etwa 3 bis etwa 300 Sekunden durchführt.

5. Verfahren nach mindestens einem der Ansprüche 2 bis 5, **dadurch gekennzeichnet, dass** man als beta-Galactosidase Enzyme aus *Aspergillus oryzae* und/oder *Bacillus circulans* einsetzt.

6. Verfahren nach mindestens einem der Ansprüche 2 bis 6, **dadurch gekennzeichnet, dass** man die Transgalactosilierung durch Zugabe von *Aspergillus oryzae* bei einer Temperatur im Bereich von etwa 50 bis etwa 60 °C und einem pH-Wert von etwa 4 bis etwa 5 durchführt.

7. Verfahren nach mindestens einem der Ansprüche 2 bis 7, **dadurch gekennzeichnet, dass** man die Transgalactosilierung durch Zugabe von *Bacillus circulans* bei einer Temperatur im Bereich von etwa 45 bis etwa 55 °C und einem pH-Wert von etwa 5,5 bis etwa 6,5 durchführt.

8. Verfahren nach mindestens einem der Ansprüche 2 bis 8, **dadurch gekennzeichnet, dass** man die Transgalactosilierung über einen Zeitraum von etwa 10 bis etwa 120 min durchführt.

9. Verwendung der Galactooligosaccharidzubereitungen nach Anspruch 1 oder erhalten oder erhältlich nach dem Verfahren nach mindestens einem der Ansprüche 2 bis 8 als Nahrungsmittel.

10. Verwendung der Galactooligosaccharidzubereitungen nach Anspruch 1 oder erhalten oder erhältlich nach dem Verfahren nach mindestens einem der Ansprüche 2 bis 8 als Nahrungsergänzungsmittel.

11. Verwendung der Galactooligosaccharidzubereitungen nach Anspruch 1 oder erhalten oder erhältlich nach dem Verfahren nach mindestens einem der Ansprüche 2 bis 8 als Futtermittel.
